(19) Europäisches Patentamt
European Patent Office
Office européen des brevets

(11) Veröffentlichungsnummer: **0 037 563**
**B1**

(12) # EUROPÄISCHE PATENTSCHRIFT

(45) Veröffentlichungstag der Patentschrift: **15.02.84**

(51) Int. Cl.³: **C 09 B 49/12**, C 09 B 55/00, C 07 D 209/88, D 06 P 1/30

(21) Anmeldenummer: **81102496.7**

(22) Anmeldetag: **02.04.81**

(54) **Schwefelfarbstoffe der Carbazolreihe, ihre Herstellung und Verwendung sowie neue Zwischenprodukte.**

(30) Priorität: **09.04.80 DE 3013625**

(43) Veröffentlichungstag der Anmeldung: **14.10.81 Patentblatt 81/41**

(45) Bekanntmachung des Hinweises auf die Patenterteilung: **15.02.84 Patentblatt 84/7**

(84) Benannte Vertragsstaaten: **CH DE FR GB IT LI NL**

(56) Entgegenhaltungen:
**DE - C - 376 815**

**CHEMICAL ABSTRACTS, Band 85, Nr. 20, November 1976, Seite 88, Nr. 144678e, Columbus, Ohio, U.S.A. T.M. KULIKOVA et al.: "Carbazole series azomethine dyes"**
**CHEMICAL ABSTRACTS, Band 78, Nr. 10, 12. März 1973, Seite 68, Nr. 59757r, Columbus, Ohio, U.S.A. T.M. KULIKOVA: "Azomethine dyes"**
***Chemical Abstracts* 74 (1971), Referat 53403t**
**Chemical Abstracts 88 (1978), Referat 50096y**

(73) Patentinhaber: **CASSELLA Aktiengesellschaft, Hanauer Landstrasse 526, D-6000 Frankfurt am Main 61 (DE)**

(72) Erfinder: **Piesch, Steffen, Dr., An der Heide 32, D-6370 Oberursel/Taunus (DE)**
Erfinder: **Weidemüller, Wolf, Dr., Nordring 101, D-6000 Frankfurt am Main 60 (DE)**

(74) Vertreter: **Urbach, Hans-Georg, Dr., Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



EP 0 037 563 B1

# Schwefelfarbstoffe der Carbazolreihe, ihre Herstellung und Verwendung sowie neue Zwischenprodukte

Die vorliegende Erfindung betrifft neue Schwefelfarbstoffe, die hergestellt werden können durch Schwefeln von Carbazolazomethinen der Formel I:

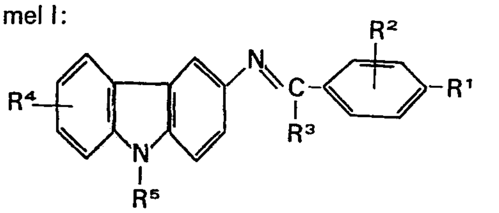

(I)

worin die Symbole $R^1$ bis $R^5$ die unten angegebenen Bedeutungen haben, nach an sich bekannten Verfahren, ihre Verwendung zum Färben von Zellulose und Polyamid sowie die zur Herstellung der neuen Farbstoffe benötigten neuen Carbazolaromethine der Formel I.

Es ist bekannt, durch Umsetzung geeigneter organischer Verbindungen mit Schwefel und ggf. Alkalisulfiden oder Polysulfiden bei erhöhter Temperatur Schwefelfarbstoffe herzustellen. Eine knappe Übersicht über die wichtigsten Gruppen bisher bekannter, geeigneter Ausgangsmaterialien findet sich beispielsweise in Kirk-Othmer, «Encyclopedia of Chemical Technology» 2. Auflage, Bd 19, Seiten 425-435. Gemäss der grossen technischen Bedeutung der Schwefelfarbstoffe existiert eine grosse Anzahl von Veröffentlichungen, die sowohl die allgemeinen Grundzüge der verschiedenen Schwefelungsverfahren, wie «Backschmelze» und «Kochschmelze» betreffen als auch einzelnen Gruppen geeigneter Ausgangsmaterialien und Schwefelfarbstoffen gewidmet sind.

Im Zusammenhang mit vorliegender Erfindung erscheinen insbesondere die Monographien:

O. Lange, «Die Schwefelfarbstoffe, ihre Herstellung und Verwendung» 2. Ausgabe, Spamer, Leipzig (1925) sowie K. Venkataraman, «The Chemistry of Synthetic Dyes and Pigments», Band 2, Academic Press, Inc., New York (1952), Kapitel 35, von Interesse.

Aus der Deutschen Patentschrift Nr. 135335 ist ein Verfahren zur Herstellung von Schwefelfarbstoffen bekannt, bei welchem aromatische Methylenamido-, Nitro-, Amido und Oxy-Benzylamido, sowie Nitro-, Amido- und Oxy-Benzylidenamidoverbindungen, und zwar solche, die die Methylen-, Benzyl- bzw. Benzylidengruppe direkt an Stickstoff gebunden enthalten, mit Schwefelalkalien und Schwefel oder Alkalilauge und Schwefel erhitzt werden.

Ein Teil der dort verwendeten Ausgangsmaterialien stellt Azomethine dar aus kernsubstituiertem Benzaldehyd und kernsubstituierten Anilinderivaten. Unter anderem wird dort auch als Tabellenbeispiel die Schwefelung von 4-(4-Nitrobenzylidenamino)-4'-nitro-diphenylamin-3'-sulfonsäure zu einem Baumwolle gelblichgrün färbenden Schwefelfarbstoff beschrieben.

Als einziger Farbstoff dieser Reihe hat das C.I. Sulphur Yellow 8 Eingang in die Technik gefunden. Dieser Farbstoff hat jedoch nur eine Lichtechtheitsnote von ca. 3 und genügt damit nicht den Anforderungen der Praxis.

Aus der Deutschen Patentschrift Nr. 165126 ist es bekannt, Amino-naphthalin-sulfonsäure-Derivate mit Benzaldehyd bzw. seinem Nitroderivat zum entsprechenden Azin zu kondensieren und anschliessend unter milden Bedingungen mit Alkalipolysulfid umzusetzen. Man erhält dabei keine Farbstoffe, sondern farblose Derivate des Naphthothiazols.

Es ist auch bereits bekannt, 3,6-Dinitrocarbazol zu schwefeln. Man erhält dabei das C.I. Sulfur Brown 6, dessen Eigenschaften wegen seiner völligen Bedeutungslosigkeit vom Color Index nicht referiert werden.

Aus einer ganzen Reihe von Patenten, z.B. der Deutschen Patentschrift Nr. 218371, dem Britischen Patent Nr. 2918/09, der US-Patentschrift Nr. 919572, sowie den betreffenden Abdrucken in «Friedländer» Band *10*, Seiten 256, 258, 71-74 und 75-81 ist es bekannt, (Leuko)-Carbazolindophenole der Formel:

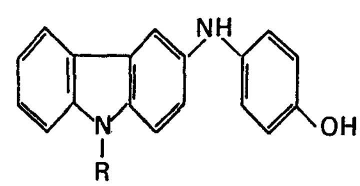

worin R Wasserstoff oder Ethyl ist, zu den blauen Schwefelfarbstoffen C.I. Sulfur Blue 42 und 43 zu schwefeln.

Weitere Derivate des Carbazols sind bislang nicht zu Schwefelfarbstoffen verarbeitet worden, obwohl die obengenannten C.I. Sulfur Blue 42 und 43 ausgezeichnete Eigenschaften haben und bereits seit 70 Jahren bekannt sind. Zieht man in Betracht, dass Farbstoffe dieses Typs auch in anderen als blauen Nuancen von grösstem Interesse gewesen wären, so erkennt man, dass alle Anstrengungen, das Gebiet der Carbazol-Schwefelfarbstoffe auszubauen, bisher gescheitert sind.

Es wurde nun gefunden, dass es gelingt, die Palette der Schwefelfarbstoffe um weitere sehr ansprechende und gesuchte gelbe, olivgrüne und braune Nuancen zu bereichern und Schwefelfarbstoffe mit für diese Klasse ausgezeichneten Echtheiten und sehr vorteilhaften Applikationsmöglichkeiten herzustellen, wenn man Carbazolazomethine der Formel I:

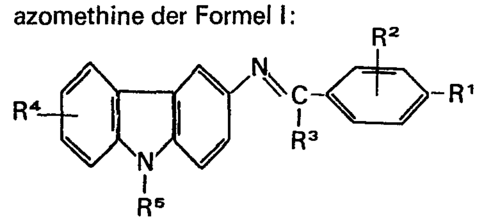

(I)

worin

$R^1$ Wasserstoff, Hydroxy, Halogen, Nitro, Alkyl mit 1 bis 8 C-Atomen, Alkoxy mit 1 bis 8 C-Atomen, Dialkylamino mit 1 bis 4 C-Atomen pro Alkylgruppe, Alkanoyloxy mit 1 bis 8 C-Atomen,

Benzoyloxy, Alkanoylamino mit 1 bis 8 C-Atomen oder Benzoylamino,

$R^2$ Wasserstoff, Hydroxy, Halogen, Alkyl mit 1 bis 8 C-Atomen oder Alkoxy mit 1 bis 8 C-Atomen,

$R^3$ Wasserstoff oder Alkyl mit 1 bis 7 C-Atomen und

$R^4$ Wasserstoff oder Halogen und

$R^5$ Wasserstoff oder Alkyl mit 1 bis 8 C-Atomen bedeuten,

nach an sich bekannten Verfahren, vorzugsweise nach dem Verfahren der «Kochschmelze» schwefelt.

Alkylreste, für die $R^1$, $R^2$, $R^3$ oder $R^5$ stehen, können linear oder verzweigt und ggf. durch ein Halogenatom, insbesondere ein Chloratom, substituiert sein. Bevorzugt sind lineare, unsubstituierte Alkylreste.

Besonders vorteilhafte Farbstoffe werden erhalten, wenn Carbazolazine der Formel I geschwefelt werden, in denen:

$R^1$ Hydroxy, Chlor, Nitro, Alkyl mit 1 bis 4 C-Atomen, Alkoxy mit 1 bis 4 C-Atomen oder Dialkylamino mit 1 oder 2 C-Atomen pro Alkylgruppe,

$R^2$ Wasserstoff, Hydroxy, Chlor, Alkyl mit 1 bis 4 C-Atomen oder Alkoxy mit 1 bis 4 C-Atomen,

$R^3$ Wasserstoff oder Alkyl mit 1 bis 3 C-Atomen, und

$R^4$ Wasserstoff,

$R^5$ Wasserstoff oder Alkyl mit 1 bis 4 C-Atomen bedeuten.

Der Substituent $R^4$ steht vorzugsweise in 6- oder 8-Stellung des Carbazolsystems.

Die Verbindungen der Formel I können auch dann zu erfindungsgemässen Schwefelfarbstoffen geschwefelt werden, wenn sie die bei der Herstellung anfallenden Isomeren, bei denen die Benzalaminogruppe in der 1-Stellung des Carbazolsystems gebunden ist, in den technisch üblichen Anteilen von bis zu ca. 25% enthalten.

Das Schwefeln der Verbindungen I in der Kochschmelze erfolgt in der an sich bekannten Weise durch Reaktion mit Schwefel und/oder Alkalipolysulfid in wässerigem Medium oder einem organischen Lösungsmittel bei erhöhter Temperatur, wie es z.B. bei O. Lange, «Die Schwefelfarbstoffe, ihre Herstellung und Verwendung» (1912), Seiten 208-220 sowie 223-225, oder in Venkataraman, «The Chemistry of Synthetic Dyes», Band 2 (1952), Seiten 1062 ff. und Seiten 1103 ff., sowie Band 7 (1974), Seiten 24 ff. Academic Press, New York, San Francisco, London beschrieben ist.

Besonders geeignet sind Kochschmelzen, die unter Verwendung von Alkalipolysulfid arbeiten, wobei pro Mol des zu schwefelnden Produkts 0,1 bis 10 Mole, vorzugsweise 0,5 bis 4 Mole Natriumsulfid und 1 bis 30 Mole, vorzugsweise 2 bis 20 Mole Schwefel eingesetzt werden. Sie werden in den für Schwefelschmelzen üblichen Lösungsmitteln durchgeführt. Als Lösungsmittel, in denen zweckmässigerweise gearbeitet werden kann, sind Alkanole oder Cycloalkanole mit 1 bis 7 C-Atomen, wie z.B. Methanol, Ethanol, Propanol, Isopropanol, n-Butanol, Isobutanol, Amylalkohol, Cyclohexanol, Methylcyclohexanol und Monoalkyläther, insbesondere Monoalkyläther von Ethylenglykol und Diethylenglykol wie z.B. Ethylenglykolmono-methyläther, -monoethyläther, -monopropyläther, Diethylenglykol-monomethyläther, mono-ethyläther und mono-propyläther geeignet.

Die Schwefelung der Verbindungen der Formel I kann unter Ausschluss von Wasser oder in rein wässerigem Medium erfolgen, wird aber normalerweise in wasserhaltigen Lösungsmitteln mit einem Wassergehalt von 5 bis 70% durchgeführt. Im Falle der mit Wasser mischbaren Lösungsmittel arbeitet man vorzugsweise unter Verwendung der üblichen hydrotropen Verbindungen, z.B. des Natriumsalzes der Xylolsulfonsäure.

Die Kochschmelze erfolgt bei Temperaturen von 80 bis 300°C, vorzugsweise 110 bis 250°C, insbesondere im Bereich von 120 bis 180°C und zweckmässigerweise bei einer Reaktionsdauer von 1 bis 100, vorzugsweise von 20 bis 80 Stunden.

Unter Inkaufnahme der bekannten Nachteile der sogenannten «Backschmelzen» oder «Schwefelschmelzen» (vgl. O. Lange, «Die Schwefelfarbstoffe» (1912) Seiten 221 und 222) können die Verbindungen der Formel I auch nach diesem Verfahren zu Schwefelfarbstoffen geschwefelt werden.

Bei der Herstellung der erfindungsgemässen Schwefelfarbstoffe durch Backschmelze werden diese mit der 7- bis 30fachen Molmenge Schwefel und der 2- bis 6fachen Molmenge Natriumsulfid 1 bis 20 Stunden auf 200 bis 300°C erhitzt. Auch hierbei kann zunächst im Lösungsmittel, vorzugsweise in Wasser, gearbeitet werden, um eine gute Homogenität des Reaktionsansatzes zu gewährleisten. Das Lösungsmittel wird dann abgedampft und die lösungsmittelfreie Schmelze zweckmässigerweise in Schutzgasatmosphäre auf die gewünschte Temperatur erwärmt.

Die nach dem erfindungsgemässen Verfahren hergestellten Schwefelfarbstoffe können auf übliche Weise isoliert und danach in die verschiedenen Handelsformen gebracht werden. Wurde die Schwefelung in einem mit Wasser mischbaren Lösungsmittel in Gegenwart von hydrotropen Verbindungen ausgeführt, so kann die Schmelze auch ohne Isolierung des Farbstoffs direkt zu flüssigen gebrauchsfertigen Farbstoffen weiterverarbeitet werden.

Die erfindungsgemäss herstellbaren Schwefelfarbstoffe lassen sich durch Reduktionsmittel, wie sie zur Reduktion von Schwefelfarbstoff üblich sind, sehr gut reduzieren und ergeben so klare wässerige Lösungen. Sie können zum Färben von vorzugsweise pflanzlichen Fasern nach den üblichen, für das Färben mit Schwefel- oder Schwefelküpenfarbstoffen bekannten Verfahren eingesetzt werden.

Hierzu werden sie mit Reduktionsmitteln, vorzugsweise Natriumdithionit, Natriumsulfid oder Natriumhydrogensulfid, möglicherweise aber auch mit Natriumformaldehyd-sulfoxylat, Glukose oder organischen Mercaptoverbindungen, wie z.B. Thioglycerin oder Thioglycolsäure, in die lösliche Leukoform überführt, die auf die Fasern aufzieht. Die in flüssiger gebrauchsfertiger Form vorliegenden Farbstoffe enthalten die lösliche Leukoform und Reduktionsmittel und können auch ohne weiteren Zusatz von Reduktionsmitteln zum Färben der genannten Fasern verwendet werden.

Nach dem Aufziehen der Farbstoffe auf die Fasern wird die Leukoform der erfindungsgemässen Schwefelfarbstoffe in üblicher Weise, beispielsweise durch «Verhängen» der Färbungen an der Luft oder Oxidation mit Oxidationsmitteln, wie beispielsweise Wasserstoffperoxid, Alkalibichromat, Alkalichlorit oder Alkalijodat wieder in die unlösliche Form des Schwefelfarbstoffs überführt. Die erfindungsgemäss herstellbaren Schwefelfarbstoffe färben die Zellulose in vollen gelben, goldbraunen und olivgrünen Tönen und stellen damit eine überaus wertvolle Ergänzung der in den coloristischen Eigenschaften vergleichbaren Carbazol-Schwefelfarbstoffen C.I. Sulfur Blue 42 und 43 dar.

Auf Zellulosefasern appliziert haben die erfindungsgemässen Farbstoffe insbesondere folgende Vorteile: sehr gute Lichtechtheit, sehr gute Nassechtheiten, sehr gute Peroxidwaschechtheit nach DIN 54015 und gute Mercerisierechtheit.

Ein weiterer Vorteil der erfindungsgemässen Farbstoffe ist ihre Stabilität gegen die Einwirkung von Alkalidithionit und Alkali-Formaldehydsulfoxylat sowie ihre einfache technische Herstellbarkeit.

Auch Polyamid lässt sich mit den erfindungsgemässen Schwefelfarbstoffen färben. Man erhält je nach Reduktionsmittel und eingesetzter Farbstoffmenge gelbe bis grünschwarze Farbtöne.

Von den zur Herstellung der erfindungsgemässen Schwefelfarbstoffen Carbazolazomethinen der Formel I ist die im Aldehydteil gänzlich unsubstituierte Verbindung der Formel Ia:

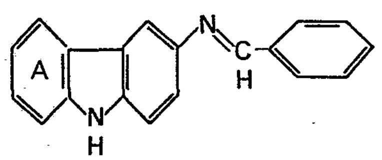

(Ia)

aus der Monographie Cohn «Die Carbazolgruppe», Seite 98, bekannt. Die gleiche Verbindung wurde auch im Hinblick auf ihr IR-Absorptionsspektrum von T.M. Kulikowa u.a. untersucht. (Structural features and chemical reactions of carbazole and its derivatives. XXXI. Synthesis of azomethines based on 3-aminocarbazole. Khim. Geterotsikl. Soedin. 1970 (10), Seiten 1353-5.) An dieser Stelle sind auch weitere Carbazolazomethine beschrieben, die in diese wissenschaftlichen Untersuchungen einbezogen worden sind.

Eine Reihe weiterer Carbazolazomethine ist von Kulikowa u.a. in «Izv. Vyssh. Uchebn. Zaved., Khim. Khim. Tekhnol.» 1977, 20 (10), Seiten 1460-3, beschrieben worden. Im Rahmen dieser Arbeit sind die Substituenteneinflüsse auf die Ionisationskonstanten von OH-substituierten Azomethinen untersucht worden.

In «Izv. Vyssh. Uchebn. Zaved.» weist Kulikowa an der oben angegebenen Stelle darauf hin, dass Koordinationsverbindungen der Carbazolazomethine mit d-Elementen technisches Interesse, beispielsweise als Katalysatoren, als Stabilisatoren oder als Reagenzien für colorimetrische oder Luminiszens-Untersuchungen, beanspruchen können. Allen an diesen Stellen beschriebenen Verbindungen ist gemeinsam, dass sie in der p-Stellung zur Azomethinbrücke im aromatischen Kern der Aldehydkomponente und im Kern A der Carbazolkomponente (siehe Formel Ia) unsubstituiert sind.

Ausserdem leiten sich diese bekannten Azomethine ausschliesslich von Aldehyden, nicht aber von Ketonen ab, so dass stets die Brücke $-N=CH-$ vorliegt.

Carbazolazomethine der Formel I, worin $R^1$ Hydroxy, Halogen, Nitro, Alkyl mit 1 bis 8 C-Atomen, Alkoxy mit 1 bis 8 C-Atomen, Dialkylamino mit 1 bis 4 C-Atomen pro Alkylgruppe, Alkanoyloxy mit 1 bis 8 C-Atomen, Benzoyloxy, Alkanoylamino mit 1 bis 8 C-Atomen oder Benzoylamino und, sofern mindestens eines der Symbole $R^3$ und $R^4$ nicht für Wasserstoff steht, auch noch Wasserstoff bedeutet, sind demnach neu.

Die neuen Carbazolazomethine der Formel I werden erhalten durch Kondensation von Derivaten des Aminocarbazols der Formel II:

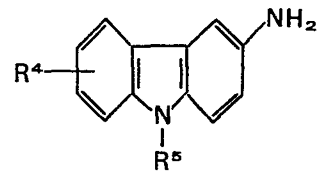

(II)

worin $R^4$ und $R^5$ die obengenannte Bedeutung haben, bzw. seiner technischen Isomerengemische mit Aldehyden oder Ketonen der Formel III:

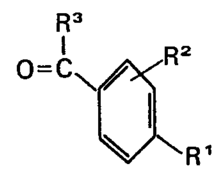

(III)

worin $R^1$, $R^2$ und $R^3$ die obengenannten Bedeutungen haben unter Wasserabspaltung.

Unter den technischen Isomerengemischen der 3-Aminocarbazole der Formel II sind solche Produkte zu verstehen, die alle bei der Herstellung der 3-Aminocarbazole im technischen Massstab entstehenden isomeren Aminocarbazole enthalten. Derartige rohe, technische Produkte enthalten neben dem Hauptprodukt, den 3-Aminocarbazolen, vorwiegend die entsprechenden 1-Aminocarbazole.

Demgemäss enthalten die erfindungsgemässen Carbazolazomethine der Formel I, die ausgehend von technischen, rohen 3-Aminocarbazolen erhalten werden, in der Regel auch Anteile der isomeren Verbindungen, die die Benzalaminogruppe in der 1-Stellung des Carbazolgerüstes enthalten.

Die Reaktionsbedingungen dieser Kondensation sind aus einer grossen Zahl von Veröffentlichungen über die Bildung von Azomethinen aus Aminen und Carbonylverbindungen bekannt. (Vgl. «Chem. Rev.» *26* (1940), Seiten 297-338;

Houben-Weyl, «Methoden der organischen Chemie», Band 7/1, Seiten 456, 457; S. Patai, «The Chemistry of the Amino Group» (1968) Interscience Publishers, Seite 352; A.E.A. Werner, «Journ. Chem. Soc.» London *23* (1944), Seite 214.)

Generell erfolgt die Kondensation, indem man die Komponenten entweder ohne Lösungsmittel oder in einem organischen Lösungsmittel so lange auf Temperaturen von 50 bis 200°C erhitzt, bis die Wasserabspaltung beendet ist. Sofern ohne Verwendung eines Lösungsmittels gearbeitet wird, ist es zweckmässig, bei Temperaturen oberhalb 90°C, insbesondere oberhalb 100°C zu arbeiten, um eine möglichst schnelle Entfernung des Reaktionswassers aus dem Reaktionssystem zu erzielen. Auf jeden Fall sollte die Temperatur so hoch gewählt werden, dass eine homogene Schmelze vorliegt.

Wird die Kondensation in einem organischen Lösungsmittel ausgeführt, so wird zweckmässigerweise beim Siedepunkt des Ansatzes gearbeitet. Hierbei kann die Kondensation begünstigt werden, wenn das abgespaltene Wasser kontinuierlich abdestilliert wird.

Als Lösungsmittel für die Durchführung der Kondensation eignen sich beispielsweise Alkanole mit 1 bis 5, vorzugsweise 3 bis 4 C-Atomen, Glycol-, Diglycol- und Triglycol-monoalkyläther, cyclische Äther wie Dioxan oder Tetrahydrofuran, oder Benzolderivate wie Toluol, Xylol, Mono- oder Dichlorbenzol.

Zur Beschleunigung der Kondensationsreaktion wird in der Regel in Gegenwart von katalytischen Mengen (0,1 bis 5%) einer anorganischen (z.B. Salzsäure) oder organischen (z.B. Ameisensäure, Essigsäure, Toluolsulfonsäure, Trifluormethansulfonsäure, Methansulfonsäure) Säure gearbeitet. Es ist jedoch auch möglich (A. Albert u.a., «Journ. of the Chemical Society», London (1943), Seite 458), die Kondensation durch Basenzusatz zu beschleunigen.

Man kann auch direkt in einer niederen Alkancarbonsäure, z.B. in Essigsäure, Propionsäure, Buttersäure, als Lösungsmittel arbeiten.

Die Verbindungen der allgemeinen Formeln II und III sind bekannte, z.T. grosstechnisch hergestellte Produkte. Sofern sie nicht handelsüblich sind, lassen sie sich nach einfachen, bekannten Verfahren gut herstellen.

Beispiele für Aminocarbazole der Formel II sind: 3-Aminocarbazol, 3-Amino-6-chlorcarbazol, 3-Amino-8-chlorcarbazol, technisches Gemisch aus 1- und 3-Aminocarbazol.

Beispiele für Aldehyde bzw. Ketone der Formel III sind: p-Hydroxybenzaldehyd; o-Hydroxybenzaldehyd; 3,4-Dihydroxybenzaldehyd; Vanillin; p-Nitrobenzaldehyd, o-, m- oder p-Methylbenzaldehyd, o-, m- oder p-Chlorbenzaldehyd, 2,4-Dichlorbenzaldehyd, p-Acetylaminobenzaldehyd, p-Hydroxyacetophenon, p-Hydroxypropionphenon, Resacatophenon, p-Dimethylaminobenzaldehyd; p-Hydroxyisocaprylophenon, 2,4-Dihydroxybutyrophenon.

Die folgenden Ausführungsbeispiele veranschaulichen die Herstellung und Verwendung der erfindungsgemässen Schwefelfarbstoffe und die Herstellung der erfindungsgemässen Zwischenprodukte der allgemeinen Formel I.

*Beispiel 1:*

a) Eine Mischung von 200 ml Diethylenglycol-mono-ethyläther, 90 g Schwefel, 71 g Natriumsulfid konz. (60%ig), 50 g xylolsulfonsäures Natrium und 50 g des Carbazolazomethins der Formel:

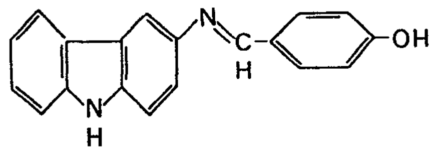

wird auf 135°C erwärmt und bei dieser Temperatur 42 Stdn. am Rückflusskühler gerührt. Danach wird die heisse Schmelze in 2 l 60°C warmes Wasser eingerührt, die erhaltene Suspension mit 100 g Natriumhydrogensulfit versetzt und 2 Std bei 70-80°C nachgerührt. Der Farbstoff wird dann abfiltriert, mit Wasser praktisch neutral gewaschen und getrocknet. Die Farbstoffausbeute beträgt 68 g. Aus der Dithionitküpe färbt der Farbstoff auf Baumwolle ein volles Gelb mit sehr guter Nass- und Lichtechtheit.

Wird die Schwefelung nicht bei 135°C, sondern bei 170°C ausgeführt, im übrigen nach dem obigen Beispiel gearbeitet, so erhält man einen Farbstoff, der Baumwolle aus der Dithionitküpe in einem schönen klaren goldbraunen Ton färbt. Die Echtheiten dieser Färbung sind ebenfalls sehr gut.

b) Das zur Herstellung des obigen Schwefelfarbstoffs eingesetzte Carbazolazomethin 3-(4-Hydroxybenzalamino)-carbazol kann wie folgt hergestellt werden:

300 g 3-Aminocarbazol, 205 g 4-Hydroxybenzaldehyd und 5 g p-Toluolsulfonsäure werden unter Rühren in 1,5 l Isopropanol eingetragen, auf ca. 85°C erhitzt und 18 Std am Rückfluss gekocht. Dann werden noch 20 g p-Hydroxybenzaldehyd zugesetzt und weitere 5 Std. am Rückfluss gekocht. Die erhaltene Reaktionsmischung wird dann auf 0°C abgekühlt, kristallisieren lassen, kalt abgesaugt und mit ca. 100 ml kaltem Isopropanol ausgewaschen. Man erhält 336 g (~ 70% d.Th.) blassgelbe Kristalle vom Schmelzpunkt 249-251°C.

Einen erfindungsgemässen Schwefelfarbstoff, der ein klares Gelbbraun färbt, erhält man, wenn man gemäss Beispiel 1a ein Azomethin schwefelt, das wie folgt erhalten wurde:

300 g rohes 3-Aminocarbazol, 205 g 4-Hydroxybenzaldehyd und 5 g p-Toluolsulfonsäure werden unter Rühren in 1,5 l Xylol eingetragen und am Wasserabscheider auf 140°C erhitzt. Bei dieser Temperatur wird 18 Std gerührt, wobei unter Sieden am Rückfluss sich im Wasserabscheider 26 ml Wasser sammeln. Es werden dann noch 20 g p-Hydroxybenzaldehyd und 1 l Xylol zugesetzt und weitere 5 Std am Wasserabscheider gerührt, wobei nochmals 3 ml Wasser abgeschieden werden. Anschliessend wird das Xylol weitgehend abdestilliert, der Rückstand mit wenig Toluol angerieben, abgesaugt und der Filterrückstand 2mal mit wenig Toluol gewaschen.

Die Rohausbeute beträgt 480 g (~ 100% d.Th.). Die Substanz schmilzt roh bei 228°C und kann ohne weitere Reinigung gemäss Abschnitt a) dieses Beispiels geschwefelt werden.

*Beispiel 2:*

a) Eine Mischung von 200 ml Diethylenglycol-mono-ethyl-äther, 90 g Schwefel, 71 g Natriumsulfid konz. (60%ig), 50 g Natrium-xylolsulfonat und 50 g des Carbazolazomethins der Formel:

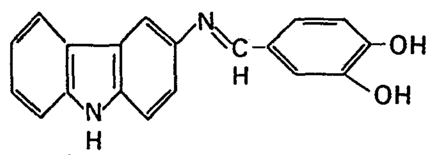

wird auf 140°C erwärmt und bei dieser Temperatur 48 Std am Rückflusskühler gerührt. Dann kühlt man das Reaktionsgemisch auf 90°C ab, verdünnt es mit 400 ml Wasser und leitet so lange Schwefeldioxid ein, bis der pH-Wert auf 2,5 gefallen ist. Man rührt noch 15 Min. bei 65°C weiter, saugt dann den Farbstoff ab, wäscht ihn mit Wasser praktisch neutral und trocknet ihn.

Die Ausbeute beträgt 68 g. Der Farbstoff färbt Baumwolle aus der Dithionitküpe in einer vollen klaren gelbgrünen Nuance in sehr guten Echtheiten, insbesondere sehr guter Waschechtheit und guter Lichtechtheit.

b) Das im Abschnitt a) als Ausgangsmaterial eingesetzte Carbazolazomethin, 3-(3,4-Dihydroxybenzalamino)-carbazol, kann analog den Angaben im Beispiel 1b) hergestellt werden, wobei die dort eingesetzten 205 + 20 g 4-Hydroxybenzaldehyd durch 232 + 22,6 g 3,4-Dihydroxybenzaldehyd ersetzt werden.

*Beispiel 3:*

a) Eine Mischung von 200 ml Diethylenglycol-mono-ethyl-äther, 108 g Schwefel, 71 g Natriumsulfid konz. (60%ig), 50 g Natrium-xylolsulfonat und 50 g des Carbazolazomethins der Formel:

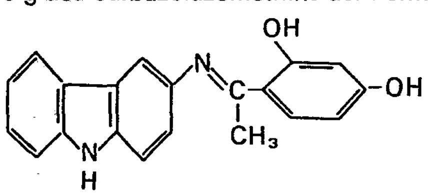

wird unter Rühren auf 135°C erwärmt und am Rückflusskühler 42 Std bei dieser Temperatur gehalten. Dann wird das Reaktionsgemisch durch Zusatz von Wasser auf ein Volumen von 1 l verdünnt und durch Einleiten von $SO_2$ der pH-Wert auf 2,3 gestellt. Anschliessend puffert man den pH-Wert durch Zusatz von Natronlauge von 38° Bé auf 4,8, filtriert den erhaltenen Farbstoff ab, wäscht ihn auf dem Filter mit Wasser neutral und trocknet ihn.

Die Farbstoffausbeute beträgt 172 g.

Der Farbstoff färbt aus der Dithionitküpe Baumwolle in einem vollen, klaren olivgrünen Ton. Die Färbung hat sehr gute Nass- und Lichtechtheiten.

b) Das zur Herstellung des obigen Schwefelfarbstoffs eingesetzte Carbazolazomethin kann wie folgt hergestellt werden:

180 g rohes 3-Aminocarbazol, 152 g Resacetophenon und 5 g p-Toluolsulfonsäure werden unter Rühren in 1,5 l Xylol eingetragen und am Wasserabscheider auf 140°C erhitzt. Bei dieser Temperatur wird unter Sieden am Rückfluss gerührt, bis sich im Wasserabscheider 18 ml Wasser abgeschieden haben. Anschliessend wird das Xylol weitgehend abdestilliert, der Rückstand mit wenig Toluol angerieben, abgesaugt und der Filterrückstand 2mal mit wenig Toluol gewaschen.

Die Rohausbeute beträgt 283 g (~ 90% d.Th.). Die Substanz schmilzt roh bei 250-265°C und kann ohne weitere Reinigung gemäss Abschnitt a) dieses Beispiels geschwefelt werden.

*Beispiel 4:*

a) Eine Mischung von 100 ml Diethylenglycol-mono-ethyläther, 35 g Schwefel, 24 g Natriumsulfid konz. (60%ig), 20 g xylolsulfonsaures Natrium und 25 g des Carbazolazomethins der Formel:

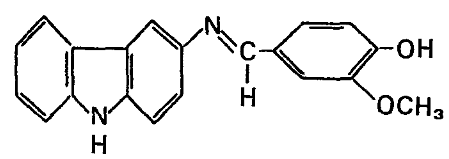

wird auf 140°C erwärmt und bei dieser Temperatur 69 Stunden am Rückflusskühler gerührt. Danach wird die heisse Schmelze in 1 l 60°C warmes Wasser eingerührt, die erhaltene Suspension mit 50 g Natriumhydrogensulfit versetzt und 2 Std bei 80°C nachgerührt. Der Farbstoff wird dann abfiltriert, mit Wasser praktisch neutral gewaschen und getrocknet. Die Farbstoffausbeute beträgt 24 g. Aus Dithionitküpe färbt der Farbstoff auf Baumwolle ein volles grünstichiges Braun mit sehr guter Nass- und Lichtechtheit.

b) Das zur Herstellung des obigen Schwefelfarbstoffs eingesetzte Carbazolazomethin, 3-(4-Hydroxy-3-methoxy-benzalamino)-carbazol, kann, wie folgt hergestellt werden:

300 g 3-Aminocarbazol, 255 g Vanillin und 5 g p-Toluolsulfonsäure werden unter Rühren in 1,5 l Xylol eingetragen und am Wasserabscheider auf 140°C erhitzt. Bei dieser Temperatur wird 18 Std gerührt, wobei unter Sieden am Rückfluss sich im Wasserabscheider 25 ml Wasser sammeln. Es werden nochmals 20 g Vanillin und 1 l Xylol zugesetzt und weitere 5 Std am Wasserabscheider gerührt, wobei nochmals 3 ml Wasser abgeschieden werden. Anschliessend wird das Xylol weitgehend abdestilliert, der Rückstand mit wenig Toluol angerieben, abgesaugt und der Filterrückstand 2 ml mit wenig Toluol gewaschen.

Die Rohausbeute beträgt 490 g (~ 94% d.Th.). Die Substanz schmilzt roh bei 180 bis 210°C und kann ohne weitere Reinigung gemäss Abschnitt a) dieses Beispiels geschwefelt werden.

*Beispiel 5:*

a) Eine Mischung von 200 ml Diethylenglycol-mono-ethyl-äther, 90 g Schwefel, 71 g Natriumsulfid konz. (60%ig), 50 g Natrium-xylolsulfonat und 50 g des Carbazolazomethins der Formel:

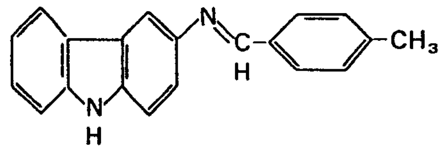

wird auf 135°C erwärmt und bei dieser Temperatur 70 Std am Rückflusskühler gerührt. Dann kühlt man das Reaktionsgemisch auf 90°C ab, verdünnt es mit Wasser auf ein Volumen von 1 l und leitet so lange Schwefeldioxid ein, bis der pH-Wert auf 3,0 gefallen ist. Man rührt noch 15 Min. bei 65°C weiter, saugt dann den Farbstoff ab, wäscht ihn mit Wasser praktisch neutral und trocknet ihn.

Die Ausbeute beträgt 53 g. Der Farbstoff färbt Baumwolle aus der Dithionitküpe in einer vollen, klaren, leicht rotstichig braunen Nuance in sehr guten Echtheiten, insbesondere sehr guter Waschechtheit und guter Lichtechtheit.

b) Das im Abschnitt a) als Ausgangsmaterial eingesetzte Carbazolazomethin, 3-(4-Methylbenzalamino)-carbazol, kann analog den Angaben in Beispiel 4b) hergestellt werden, wobei die dort eingesetzten 255 + 20 g Vanillin durch 201 + 16 g 4-Methyl-benzaldehyd ersetzt werden.

*Beispiel 6:*

a) Eine Mischung von 200 ml Diethylenglycolmono-ethyläther, 90 g Schwefel, 71 g Natriumsulfid konz. (60%ig), 50 g xylolsulfonsaures Natrium und 50 g des Carbazolazomethins der Formel:

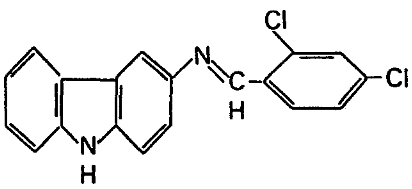

wird auf 135°C erwärmt und bei dieser Temperatur 48 Std. am Rückflusskühler gerührt. Danach wird die heisse Schmelze in 2 l 60°C warmes Wasser eingerührt, die erhaltene Suspension mit 100 g Natriumhydrogensulfit versetzt und 2 Std bei 70-80°C nachgerührt. Der Farbstoff wird dann abfiltriert, mit Wasser praktisch neutral gewaschen und getrocknet. Die Farbstoffausbeute beträgt 68 g. Aus Dithionitküpe färbt der Farbstoff auf Baumwolle ein volles Goldgelb mit sehr guter Nass- und Lichtechtheit.

b) Das zur Herstellung des obigen Schwefelfarbstoffs eingesetzte Carbazolazomethin, 3-(2,4-Dichlorbenzalamino)-carbazol, kann wie folgt hergestellt werden:

450 ml Isopropanol, 135 g rohes 3-Aminocarbazol, 134 g 2,4-Dichlorbenzaldehyd und 5 ml Ameisensäure konz. werden 8 Std unter Rühren am Rückfluss gekocht. Nach dem Abkühlen wird die Suspension abgesaugt, der Filterrückstand mit ca. 100 ml Isopropanol gewaschen und getrocknet. Man erhält 120 g (~ 50% d.Th.) des für die Schwefelung hinreichend reinen Produkts vom Schmelzpunkt ca. 250°C.

Aus der Mutterlauge können noch weitere Mengen der Substanz isoliert werden.

Zum gleichen Carbazolazomethin gelangt man nach folgendem Verfahren:

300 g rohes 3-Aminocarbazol, 294 g 2,4-Dichlorbenzaldehyd und 5 g p-Toluolsulfonsäure werden unter Rühren in 1,5 l Xylol eingetragen und am Wasserabscheider auf 140°C erhitzt. Bei dieser Temperatur wird 18 Std gerührt, wobei unter Sieden am Rückfluss sich im Wasserabscheider 28 ml Wasser sammeln. Anschliessend wird das Xylol weitgehend abdestilliert, der Rückstand mit wenig Toluol angerieben, abgesaugt und der Filterrückstand 2mal mit wenig Toluol gewaschen.

Die Rohausbeute beträgt 510 g (~ 91% d.Th.), schmilzt roh unscharf bei 225°C und kann ohne weitere Reinigung gemäss Abschnitt a) dieses Beispiels geschwefelt werden.

*Beispiel 7:*

a) In einer Mischung von 50 g 31%iger wässeriger Natriumhydrogensulfidlösung, 20 g Natriumxylolsulfonat und 200 g Diethylenglycol-monoethyläther werden bei 25-26°C unter Rühren 50 g des Carbazolazomethins der Formel:

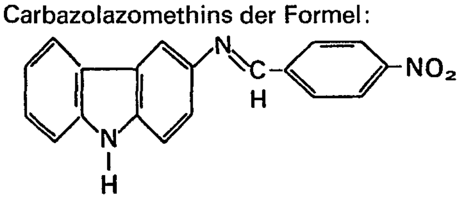

eintragen. Innerhalb weniger Minuten steigt die Temperatur des Ansatzes von selbst auf 76°C. Man rührt 1 Std bei dieser Temperatur nach und setzt dann 108 g Schwefel und 42 g Natriumsulfid konz. (60%ig) zu. Danach wird unter Rühren auf 140°C erwärmt und 60 Std bei dieser Temperatur am Rückfluss weitergerührt. Anschliessend wird die heisse Schmelze in 2 l 60°C warmes Wasser eingerührt, die erhaltene Suspension mit 100 g Natriumhydrogensulfit versetzt und 2 Std bei 80°C nachgerührt. Der Farbstoff wird dann abfiltriert, mit Wasser praktisch neutral gewaschen und getrocknet. Die Farbstoffausbeute beträgt 77 g. Aus Dithionitküpe färbt der Farbstoff auf Baumwolle ein volles, klares gelbstichiges Braun mit sehr guter Nass- und Lichtechtheit.

Wird analog der vorstehenden Vorschrift anstelle von 50 g des obigen Carbazolazomethins die gleiche Menge des 9-Ethylderivats der Formel:

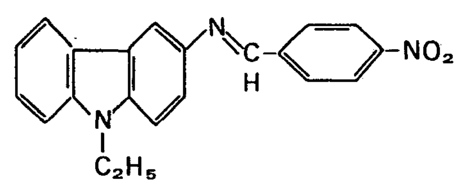

eingesetzt und die Schwefelung bei 160°C durchgeführt, so werden 62 g eines Schwefelfarbstoffs erhalten, der auf Baumwolle ein neutrales, klares Braun färbt.

b) Das zur Herstellung des obigen Schwefelfarbstoffs benötigte Carbazolazomethin 3-(4-Nitrobenzalamino)-carbazol wird wie folgt hergestellt:

Eine Mischung von 1,5 l Ethylenglycol-monoethyläther, 300 g 3-Aminocarbazol, 250 g p-Nitrobenzaldehyd und 15 ml Ameisensäure wird unter Rühren 4 Std am Rückfluss gekocht. Dann wird das Lösungsmittel am Rotationsverdampfer abgetrieben, der Rückstand mit 1 l Methanol aufgekocht und die Suspension abgesaugt. Der Filterrückstand wird mit ca. 100 ml Methanol nachgewaschen und getrocknet. Rote Kristalle vom Schmelzpunkt 198-200°C, Ausbeute 360 g (~ 70% d.Th.).

Das 9-Ethylderivat wird erhalten, wenn anstelle der 300 g 3-Aminocarbazol 346 g 3-Amino-9-ethylcarbazol nach der obigen Vorschrift mit p-Nitrobenzaldehyd umgesetzt werden. Die Ausbeute beträgt 85% d.Th.

Zu erfindungsgemässen, wertvollen Schwefelfarbstoffen gelangt man auch, wenn man die folgenden Carbazolazomethine analog den vorstehenden Ausführungsbeispielen herstellt und schwefelt.

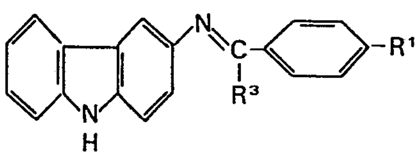

$R^1 = -COOCH_3$; $R^3 = H$
$R^1 = -OH$; $R^3 = -C_3H_7$
$R^1 = -OH$; $R^3 = -C_8H_{17}$ (iso)
$R^1 = -N(CH_3)_2$; $R^3 = H$

## Patentansprüche

1. Schwefelfarbstoffe, herstellbar durch Schwefeln von Carbazolazomethinen der Formel I:

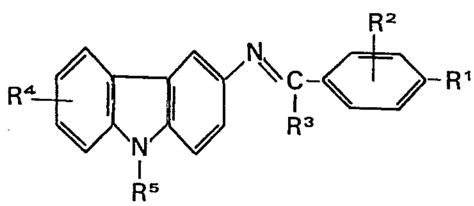 (I)

worin:

$R^1$ Wasserstoff, Hydroxy, Halogen, Nitro, Alkyl mit 1 bis 8 C-Atomen, Alkoxy mit 1 bis 8 C-Atomen, Dialkylamino mit 1 bis 4 C-Atomen pro Alkylgruppe, Alkanoyloxy mit 1 bis 8 C-Atomen, Benzoyloxy, Alkanoylamino mit 1 bis 8 C-Atomen oder Benzoylamino,

$R^2$ Wasserstoff, Hydroxy, Halogen, Alkyl mit 1 bis 8 C-Atomen oder Alkoxy mit 1 bis 8 C-Atomen,

$R^3$ Wasserstoff oder Alkyl mit 1 bis 7 C-Atomen bedeuten,

$R^4$ Wasserstoff oder Halogen,

$R^5$ Wasserstoff oder Alkyl mit 1 bis 8 C-Atomen, bedeuten,

bei der Zerstellung anfallenden Isomeren, bei denen die Benzolaminogruppe in der 1-Stellung des Carbazolsystems gebunden ist, in den technisch üblichen Anteilen von bis zu ca. 25% enthalten können, nach an sich bekannten Verfahren.

2. Schwefelfarbstoffe gemäss Anspruch 1, wobei im Carbazolazomethin der Formel I:

$R^1$ Hydroxy, Chlor, Nitro, Alkyl mit 1 bis 4 C-Atomen, Alkoxy mit 1 bis 4 C-Atomen oder Dialkylamino mit 1 oder 2 C-Atomen pro Alkylgruppe,

$R^2$ Wasserstoff, Hydroxy, Chlor, Alkyl mit 1 bis 4 C-Atomen, oder Alkoxy mit 1 bis 4 C-Atomen,

$R^3$ Wasserstoff oder Alkyl mit 1 bis 3 C-Atomen,

$R^4$ Wasserstoff,

$R^5$ Wasserstoff oder Alkyl mit 1 bis 4 C-Atomen bedeutet.

3. Schwefelfarbstoffe gemäss den Ansprüchen 1 und 2, herstellbar durch Schwefeln der Verbindungen der Formel I nach dem an sich bekannten Verfahren der «Kochschmelze».

4. Verfahren zur Herstellung von Schwefelfarbstoffen durch Umsetzung geeigneter Ausgangsmaterialien mit Schwefel und Alkalisulfid oder Alkaliopolysulfid nach den an sich bekannten Verfahren der «Kochschmelze» oder der «Backschmelze», dadurch gekennzeichnet, dass als geeignete Ausgangsmaterialien Carbazolazomethine der Formel I des Anspruchs 1 eingesetzt werden.

5. Carbazolazomethine der Formel I:

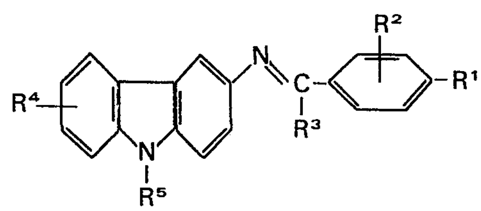 (I)

worin $R^1$ Hydroxy, Halogen, Nitro, Alkyl mit 1 bis 8 C-Atomen, Alkoxy mit 1 bis 8 C-Atomen, Dialkylamino mit 1 bis 4 C-Atomen pro Alkylgruppe, Alkanoyloxy mit 1 bis 8 C-Atomen, Benzoyloxy, Alkanoylamino mit 1 bis 8 C-Atomen oder Benzoylamino und, sofern mindestens eines der Symbole $R^3$ und $R^4$ nicht für Wasserstoff steht, auch noch Wasserstoff,

$R^2$ Wasserstoff, Hydroxy, Halogen, Alkyl mit 1 bis 8 C-Atomen oder Alkoxy mit 1 bis 8 C-Atomen,

$R^3$ Wasserstoff oder Alkyl mit 1 bis 7 C-Atomen und

$R^4$ Wasserstoff, oder Halogen,

$R^5$ Wasserstoff oder Alkyl mit 1 bis 8 C-Atomen bedeuten.

6. Carbazolazomethine der Formel I, dadurch gekennzeichnet, dass

$R^1$, $R^2$, $R^3$, $R^4$ und $R^5$ die in Anspruch 2 angegebenen Bedeutungen haben.

7. Verfahren zur Herstellung der Carbazolazomethine der Formel I:

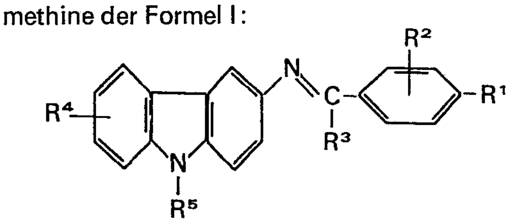 (I)

worin

$R^1$, $R^2$, $R^3$, $R^4$ und $R^5$ die in Anspruch 5 angegebenen Bedeutungen haben, dadurch gekennzeichnet, dass ein Derivat des Aminocarbazols der Formel II:

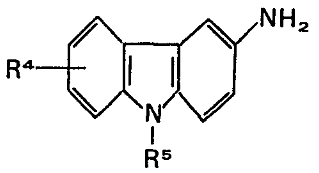

(II)

worin

$R^4$ und $R^5$ die obengenannte Bedeutung hat, welches auch sein bei der Zerstellung anfallendes Isomeres, bei dem die Aminogruppe in der 1-Stellung des Carbazolsystemes gebunden ist, in den technisch üblichen Anteilen von bis zu ca. 25% enthalten kann, mit Aldehyden oder Ketonen der Formel III:

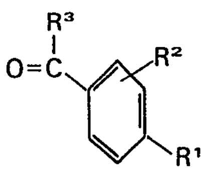

(III)

worin

$R^1$, $R^2$ und $R^3$ die obengenannten Bedeutungen haben, unter Wasserabspaltung in an sich bekannter Weise kondensiert wird.

8. Verfahren gemäss Anspruch 7, dadurch gekennzeichnet, dass die Kondensation in einem organischen Lösungsmittel unter Säurekatalyse vorgenommen wird.

9. Verwendung der Schwefelfarbstoffe der Ansprüche 1 bis 3 zum Färben von Zellulose oder Polyamid.

**Claims**

1. Sulphur dyestuffs which can be prepared by sulphurising, in accordance with processes which are in themselves known, carbazoleazomethines which have the formula I:

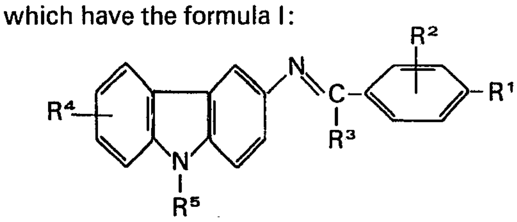

(I)

wherein:

$R^1$ denotes hydrogen, hydroxyl, halogen, nitro, alkyl having 1 to 8 C atoms, alkoxy having 1 to 8 C atoms, dialkylamino having 1 to 4 C atoms per alkyl group, alkanoyloxy having 1 to 8 C atoms, benzoyloxy, alkanoylamino having 1 to 8 C atoms or benzoylamino,

$R^2$ denotes hydrogen, hydroxyl, halogen, alkyl having 1 to 8 C atoms or alkoxy having 1 to 8 C atoms,

$R^3$ denotes hydrogen or alkyl having 1 to 7 C atoms,

$R^4$ denotes hydrogen or halogen, and

$R^5$ denotes hydrogen or alkyl having 1 to 8 C atoms, and which can also contain the isomers formed in the course of their preparation and in which the benzalamino group is bound in the 1-position of the carbazole system, in technically customary proportions of up to approximately 25%.

2. Sulphur dyestuffs according to Claim 1, wherein, in the carbazoleazomethine of the formula I,

$R^1$ denotes hydroxyl, chlorine, nitro, alkyl having 1 to 4 C atoms, alkoxy having 1 to 4 C atoms or dialkylamino having 1 or 2 C atoms per alkyl group,

$R^2$ denotes hydrogen, hydroxyl, chlorine, alkyl having 1 to 4 C atoms or alkoxy having 1 to 4 C atoms,

$R^3$ denotes hydrogen or alkyl having 1 to 3 C atoms,

$R^4$ denotes hydrogen, and

$R^5$ denotes hydrogen or alkyl having 1 to 4 C atoms.

3. Sulphur dyestuffs according to Claims 1 and 2 which can be prepared by sulphurising the compounds of the formula I in accordance with the «boil-melt» process, which is in itself known.

4. Process for the manufacture of sulphur dyestuffs by reacting suitable starting materials with sulphur and an alkali metal sulphide or an alkali metal polysulphide in accordance with the «boil-melt» or «bake-melt» processes, which are in themselves known, characterised in that carbazoleazomethines of the formula of claim 1 are employed as starting materials.

5. Carbazoleazomethines of the formula I:

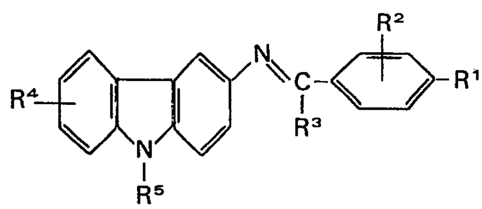

(I)

wherein:

$R^1$ denotes hydroxyl, halogen, nitro, alkyl having 1 to 8 C atoms, alkoxy having 1 to 8 C atoms, dialkylamino having 1 to 4 C atoms per alkyl group, alkanoyloxy having 1 to 8 C atoms, benzoyloxy, alkanoylamino having 1 to 8 C atoms or benzoylamino and $R^1$ also denotes hydrogen, if at least one of the symbols $R^3$ and $R^4$ does not represent hydrogen,

$R^2$ denotes hydrogen, hydroxyl, halogen, alkyl having 1 to 8 C atoms or alkoxy having 1 to 8 C atoms,

$R^3$ denotes hydrogen or alkyl having 1 to 7 C atoms,

$R^4$ denotes hydrogen or halogen, and

$R^5$ denotes hydrogen or alkyl having 1 to 8 C atoms.

6. Carbazoleazomethines of the formula I, characterised in that $R^1$, $R^2$, $R^3$, $R^4$ and $R^5$ have the meanings indicated in Claim 2.

7. Process for the manufacture of carbazoleazomethines of the formula I:

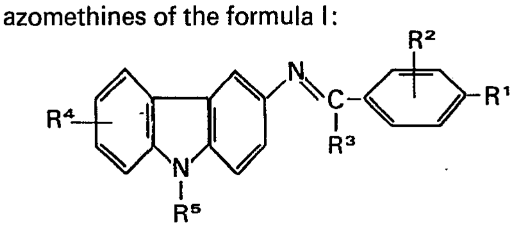

(I)

wherein:

$R^1$, $R^2$, $R^3$, $R^4$ and $R^5$ have the meanings indicated in Claim 5, characterised in that a derivative of an aminocarbazole of the formula II:

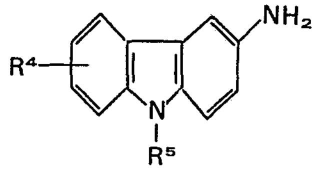

(II)

wherein:

$R^4$ and $R^5$ have the above-mentioned meaning, which can also contain, in technically customary proportions of up to approximately 25%, its isomer formed in the course of its preparation, in which isomer the amino group can be bound in the 1-position of the carbazole system, is subjected, in a manner which is in itself known, to a condensation reaction, with elimination of water, with aldehydes or ketones of the formula III:

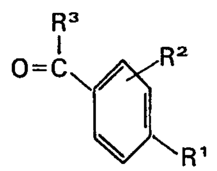

(III)

wherein:

$R^1$, $R^2$ and $R^3$ have the above-mentioned meanings.

8. Process according to Claim 7, characterised in that the condensation reaction is carried out in an organic solvent with acid catalysis.

9. Use of the sulphur dyestuffs of Claims 1 to 3 for dyeing cellulose or polyamide.

**Revendications**

1. Colorants au soufre, pouvant être préparés suivant des procédés connus par sulfuration de composés carbazolazaméthiniques de formule I:

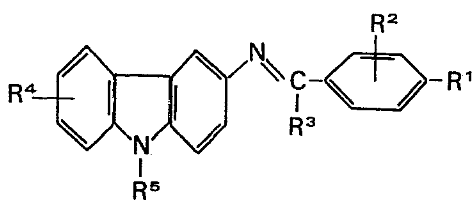

(I)

dans laquelle:

$R^1$ représente l'hydrogène, un hydroxyle, un halogène, le groupe nitro, un alkyle en $C_1$ à $C_8$, un alcoxy en $C_1$ à $C_8$, un groupe dialkylamino en $C_1$ à $C_4$ par groupe alkyle, un groupe alcanoyloxy en $C_1$ à $C_8$, benzoyloxy, alcanoylamino en $C_1$ à $C_8$ ou benzoylamino,

$R^2$ représente l'hydrogène, un hydroxyle, un halogène, un alkyle ou un alcoxy en $C_1$ à $C_8$,

$R^3$ l'hydrogène ou un alkyle en $C_1$ à $C_7$,

$R^4$ l'hydrogène ou un halogène, et

$R^5$ l'hydrogène ou un alkyle en $C_1$ à $C_8$, et qui peuvent encore contenir les isomères formés au cours de leur préparation, isomères dans lesquels le groupe benzalamino occupe la position 1 du cycle de carbazole, dans les proportions techniquement habituelles pouvant s'élever jusqu'aux environs de 25%.

2. Colorants au soufre selon la revendication 1, dans lesquels, dans la carbazolazométhine de formule I,

$R^1$ est un hydroxyle, un atome de chlore, un groupe nitro, un alkyle en $C_1$ à $C_4$, un alcoxy en $C_1$ à $C_4$ ou un groupe dialkylamino en $C_1$ ou $C_2$ dans le groupe alkyle,

$R^2$ est l'hydrogène, un hydroxyle, un atome de chlore, ou un alkyle en $C_1$ à $C_4$ ou un alcoxy en $C_1$ à $C_4$,

$R^3$ est l'hydrogène ou un alkyle en $C_1$ à $C_3$,

$R^4$ l'hydrogène, et

$R^5$ l'hydrogène ou un alkyle en $C_1$ à $C_4$.

3. Colorants au soufre selon la revendication 1 et 2 pouvant être préparés par sulfuration des composés de formule I par le procédé connu dit «fusion-ébullition».

4. Procédé de préparation de colorants au soufre par réaction de matières de départ appropriées avec du soufre et un sulfure ou un polysulfure de métal alcalin suivant les procédés connus dits de «fusion-ébullition» ou de «fusion-cuisson», procédé caractérisé en ce que l'on part de composés carbazolazométhiniques de formule I selon la revendication 1.

5. Les composés carbazolazométhiniques (azaméthiniques) de formule I:

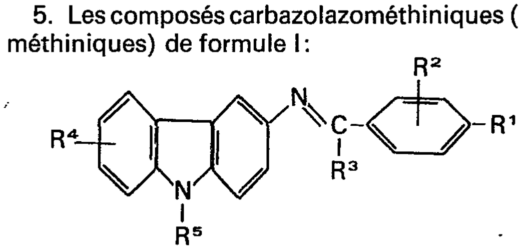

(I)

dans lesquels:

$R^1$ est un hydroxyle, un halogène, le groupe nitro, un alkyle en $C_1$ à $C_8$, un alcoxy en $C_1$ à $C_8$, un dialkylamino en $C_1$ à $C_4$ par groupe alkyle, un alcanoyloxy en $C_1$ à $C_8$, un benzoyloxy, un alcanoylamino en $C_1$ à $C_8$ ou un benzoylamino, ou encore l'hydrogène si, parmi $R^3$ et $R^4$, l'un des deux au moins n'est pas l'hydrogène,

$R^2$ est l'hydrogène, un hydroxyle, un halogène, un alkyle en $C_1$ à $C_8$ ou un alcoxy en $C_1$ à $C_8$,

$R^3$ est l'hydrogène ou un alkyle en $C_1$ à $C_7$,

$R^4$ l'hydrogène ou un halogène, et

$R^5$ l'hydrogène ou un alkyle en $C_1$ à $C_8$.

6. Composés carbazolazométhiniques de formule I, caractérisés en ce que les symboles $R^1$, $R^2$, $R^3$, $R^4$ et $R^5$ ont les significations données à la revendication 2.

7. Procédé de préparation des composés carbazolazométhiniques de formule I:

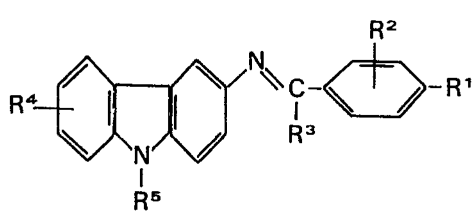

(I)

dans lesquels $R^1$, $R^2$, $R^3$, $R^4$ et $R^5$ ont les significations données à la revendication 5, procédé caractérisé en ce que l'on condense de manière connue, avec élimination d'eau, un dérivé d'aminocarbazole de formule II:

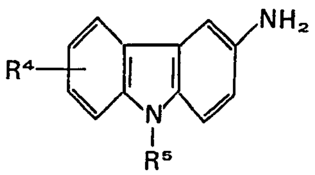

(II)

($R^4$ et $R^5$ ayant les significations indiquées, et qui peut contenir aussi son isomère formé au cours de sa préparation, dans lequel le groupe amino est lié à la position 1 du cycle de carbazole, dans les proportions techniquement habituelles pouvant s'élever jusqu'aux environs de 25%) avec des aldéhydes ou des cétones de formule III:

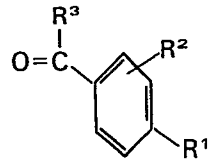

(III)

les symboles $R^1$, $R^2$ et $R^3$ ayant les significations données.

8. Procédé selon la revendication 7, caractérisé en ce que la condensation se fait dans un solvant organique, catalysée par un acide.

9. L'emploi des colorants au soufre selon l'une quelconque des revendications 1 à 3 pour la teinture de cellulose ou de polyamides.